# FASCICULE DE BREVET EUROPEEN

(11) **EP 0 597 751 B1**
(45) Date de publication et mention de la délivrance du brevet: **21.01.1998**
(21) Numéro de dépôt: 93402651.9
(22) Date de dépôt: 28.10.1993
(51) Int. Cl.: C07C 63/26, C07C 51/09, C07C 27/02

(54) **Procédé amélioré de récupération de téréphtalate de métal alcalin ou alcalino-terreux et d'alkylèneglycol à partir de polytéréphtalates d'alkylène**
Verfahren zur Rückgewinnung von Alkalimetall- oder Erdalkalimetallterephthalat und Alkylenglykol aus Alkylenpolyterephthalaten
Process for recovery of alkali metal or alkali earth metal terephtalate and alkylene glycol from alkylene polyterephtalates

(30) Priorité: 09.11.1992 FR 9213583
(43) Date de publication de la demande: 18.05.1994
(73) Titulaire: INSTITUT FRANCAIS DU PETROLE, 92502 Rueil-Malmaison (FR); Benzaria, Jacques Raphäel, 60230 Chambly (FR)
(72) Inventeur: Benzaria, Jacques, F-60230 Chambly (FR); Durif-Varambon, Bruno, F-38200 Vienne (FR); Dawans, François, F-78380 Bougival (FR); Gaillard, Jean-Bernard, F-38400 Saint Martin d'Heres (FR)
(74) Mandataire: Andreeff, François

(56) Documents cités:
- EP-A- 0 037 984
- EP-A- 0 497 662
- WO-A-91/09004
- DE-A- 2 802 125
- FR-A- 2 342 152
- GB-A- 1 130 695

## Description

L'invention concerne un procédé amélioré de récupération de téréphtalate de métal alcalin ou alcalino-terreux ou d'acide téréphtalique et d'alkylèneglycol par saponification d'un polytéréphtalate d'alkylène. Elle concerne plus particulièrement la fabrication en continu de téréphtalates de métaux alcalins ou alcalino-terreux à partir de polytéréphtalates d'alkylène.

Elle permet d'améliorer le recyclage des déchets de polytéréphtalates d'alkylène provenant par exemple de films, de fibres ou de bouteilles usagées. Le procédé de l'invention permet en outre de récupérer l'alkylèneglycol avec une bonne pureté, pour sa réutilisation.

La saponification alcaline de polytéréphtalate d'alkylène en solution est décrite dans l'art antérieur (voir, par exemple, les brevets US-A-3.317.519 et US-A- 3.544.622); elle s'effectue généralement en solution diluée dans l'eau ou dans le glycol, en général sous pression.

La demande internationale WO-A-9109004 décrit notamment la dépolymérisation de polyesters au moyen d'un alcoxyde, ou d'un hydroxyde alcalin ou alcalino-terreux, en présence d'alcool ou de glycol et d'au moins un solvant aprotique polaire tel que la N-méthyl-pyrrolidone ou le diméthylsulfoxyde.

Dans la demande de brevet FR-A-2.672.049, les déposants ont décrit un procédé de fabrication de téréphtalate de métal alcalin ou alcalino-terreux par chauffage de polytéréphtalate d'alkylène avec un hydroxyde de métal alcalin ou alcalino-terreux, en l'absence ou en présence d'eau.

On a maintenant découvert qu'il était possible de réaliser la saponification par un hydroxyde alcalin ou alcalino-terreux, de polytéréphtalate d'alkylène sans solvant ni diluant, à l'état fondu, tout en éliminant l'alkylène-glycol formé, le téréphtalate alcalin ou alcalino-terreux récupéré étant obtenu sous la forme d'une poudre.

De manière inattendue, on a découvert que la réaction de saponification entre un polytéréphtalate d'alkylène et un hydroxyde alcalin ou alcalino-terreux, amorcée par chauffage préalable du mélange des réactifs à une température de 60 à 250°C se poursuivait d'elle-même, sans chauffage supplémentaire. Le polytéréphtalate continue à réagir jusqu'à la fin de la réaction, même sans agitation, pour former un produit soluble dans l'eau, sans aucune trace du polymère de départ.

Le principal avantage du procédé de l'invention est qu'il permet de produire, en continu, un composé de téréphtalate alcalin ou alcalino-terreux sous forme poudreuse et d'éliminer le stade de la récupération de l'alkylèneglycol dans les eaux de purification. Le solide poudreux obtenu est facile à stocker, à transporter et à redissoudre ultérieurement en phase aqueuse, en vue de sa purification et le cas échéant de sa transformation en acide ou ester téréphtalique.

L'élimination de l'alkylèneglycol sous forme gazeuse préalablement à la dissolution du téréphtalate de métal alcalin facilite la purification de ce dernier par désolvatation en particulier de certains colorants et produits solubles dans l'alkylèneglycol.

Dans l'art antérieur, les glycols issus de la saponification étaient soit extraits de la solution à l'aide d'un solvant, après filtration de l'acide téréphtalique, soit relargués par saturation de la solution par augmentation de la salinité du milieu ("salting out"), ou encore envoyés à la station d'épuration. Ces méthodes correspondaient à une opération supplémentaire coûteuse en réactifs, en matériel et en temps. Le procédé de l'invention permet d'éliminer le glycol formé sans apport de réactif supplémentaire, soit par entraînement au moyen d'un gaz inerte, soit par extraction sous pression réduite, au cours de la réaction de saponification, ce qui simplifie le schéma de la fabrication du téréphtalate du métal alcalin ou alcalino-terreux recherché.

Vis-à-vis de l'art antérieur, le procédé selon l'invention permet de traiter des déchets de polyesters, dans de petites unités industrielles simples et délocalisées et de rassembler ensuite les produits de saponification, constitués essentiellement de téréphtalates mono et di-alcalins ou alcalino-terreux issus de diverses provenances, pour les traiter dans une même installation industrielle, en vue de les purifier et de les transformer en acide ou ester téréphtalique réutilisable en polycondensation. Une telle opération réalisée en deux étapes distinctes permet de réduire les investissements et les frais de collecte et transport des déchets de plastiques.

Le procédé selon l'invention permet d'obtenir industriellement, et de manière plus économique, des téréphtalates alcalins ou alcalino-terreux à partir desquels on peut former de l'acide ou un ester téréphtalique de grande pureté. Cette technique s'avère particulièrement intéressante pour le recyclage de films, de fibres ou de bouteilles en polytéréphtalate d'éthylène (PET).

Dans son principe, le procédé selon la présente invention consiste à réaliser la saponification par un hydroxyde alcalin ou alcalino-terreux, sans solvant ni diluant, à l'état fondu, la réaction étant amorcée par passage en continu d'un mélange solide de polytéréphtalate et d'hydroxyde de métal alcalin ou alcalino-terreux dans un malaxeur-extrudeur chauffé, spécialement équipé pour l'élimination de vapeurs, et/ou étant poursuivie en dehors de ce premier malaxeur-extrudeur, dans une enceinte contrôlée thermiquement, agitée et maintenue sous pression réduite pour éliminer le glycol formé, le glycol pouvant aussi être entraîné par un courant de gaz inerte , mais en opérant à une température plus élevée.

D'une manière générale, le procédé de l'invention peut être défini par le fait que
a) on mélange la composition de départ comprenant principalement du polytéréphtalate d'alkylène avec un hydroxyde de métal alcalin ou alcalino-terreux pratiquement anhydre en l'absence de solvant;
b) on chauffe le mélange obtenu, ledit mélange étant au moins en partie à l'état fondu, de manière à amorcer la saponification du polytéréphtalate d'alkylène et à en permettre la poursuite sans chauffage ultérieur;
c) on élimine l'alkylèneglycol formé au cours de la réaction de saponification sous forme gazeuse; et
d) on recueille le téréphtalate de métal alcalin ou alcalino-terreux sous la forme d'un produit poudreux issu de la réaction de saponification.

Le procédé de l'invention est décrit de façon plus détaillée ci-après.

Le polytéréphtalate d'alkylène, consistant par exemple en des déchets de films, de fibres ou de bouteilles, est préalablement broyé ou déchiqueté et mélangé intimement avec un hydroxyde de métal alcalin ou alcalino-terreux sous forme de poudre, de granulés, de copeaux ou de pastilles, ces réactifs étant dosés de manière que le réactif alcalin ou alcalino-terreux soit en proportion stoechiométrique ou en léger excès, par rapport aux groupements carboxyliques du polytéréphtalate d'alkylène. Les réactifs sont introduits, par exemple par gravité, dans un réacteur qui peut être un malaxeur-extrudeur opérant en continu.

Dans le cas d'un malaxeur-extrudeur en continu, l'introduction des réactifs au début de la chambre de malaxage permet de bénéficier de toute la longueur du temps de séjour dans le malaxeur. Sans sortir du cadre de l'invention, il est également possible d'introduire plus tard ou en divers endroits tout ou partie des réactifs.

Les débits d'introduction sont variables et fonction des conditions de réaction, des dimensions et du type de malaxeur-extrudeur et du temps de séjour des réactifs requis pour amorcer la saponification. En général, ils sont compris entre 50 et 10.000 kg/h sur un malaxeur-extrudeur de 5 pouces X 36 pouces pris comme référence.

C'est en utilisant l'effet d'hystérésis qu'on obtient un avantage supplémentaire du procédé de la présente invention, de pouvoir atteindre des débits très élevés, dans un réacteur malaxeur-extrudeur de petit volume, en amorçant la saponification et en induisant la poursuite de la réaction jusqu'à son achèvement, celle ci pouvant s'effectuer ensuite dans des équipements simplifiés grâce à l'exploitation de l'exothermicité de la réaction.

Le mélange et l'homogénéisation des réactifs nécessaires pour amorcer la saponification s'effectuent à une température comprise entre 60 et 250°C, et de préférence entre 100° et 200°C et plus spécifiquement entre 120° et 160°C, afin d'éviter la formation de produits secondaires de dégradation.

Plus particulièrement, la réaction se déroule avec une vitesse satisfaisante aux environs de 150°C et il est préférable de contrôler la température par circulation de fluide refroidi. Le réacteur est alimenté par un mélange de polytéréphtalate d'alkylène (en particulier de P.E.T.) et d'hydroxyde (en particulier de soude), à l'aide d'un doseur.

Le mélange dans le réacteur-malaxeur-extrudeur peut être réalisé par divers arrangements des palettes le long de deux arbres parallèles tournant dans le même sens ou non au sein d'un malaxeur-extrudeur à double vis. En général, un choix judicieux de l'arrangement des palettes comporte, d'une part, à l'entrée de la chambre de malaxage, des vis sans fin autonettoyantes, assurant l'introduction des réactifs alimentés par gravité, d'autre part, plusieurs types de palettes de mélange, d'avance, de cisaillement et de retard en vue de disposer d'une capacité optimale de mélange, homogénéisation et malaxage adaptés au procédé de saponification.

Selon un mode préféré de mise en oeuvre de l'invention, on amorce la saponification en associant un chauffage et l'effet d'hystérésis sur le mélange de polymère et d'hydroxyde alcalin ou alcalino-terreux. Selon l'importance d'hystérésis, la température du mélange est augmentée par le travail mécanique, ce qui permet d'utiliser l'énergie provoquée par le travail de la matière et de réduire le temps de séjour dans le réacteur-malaxeur-extrudeur.

Selon une variante du procédé, l'alkylène-glycol est éliminé en continu du réacteur-malaxeur-extrudeur sous pression réduite et/ou par entraînement au moyen d'un gaz inerte, qui peut être l'azote, puis condensé par refroidissement.

Selon une autre variante du procédé, à la sortie du réacteur malaxeur-extrudeur, le mélange réactionnel est dégazé, le glycol étant éliminé sous pression réduite et/ou par entraînement au moyen d'un courant de gaz inerte; les vapeurs du glycol formé sont alors condensées. Cette seconde étape du procédé de l'invention est réalisée, de préférence, en maintenant le mélange issu du malaxeur-extrudeur à une température comprise entre 50° et 200°C durant un temps suffisant pour achever la saponification, par exemple de 5 à 30 minutes. Cette étape peut être réalisée en faisant passer le mélange dans un réacteur à simple vis ou sur un tapis chauffant ou encore dans une enceinte permettant le maintien en température et la récupération du glycol formé.

A titre d'illustration, la sortie du réacteur-malaxeur-extrudeur communique avec une chambre chauffante étanche munie d'une double vis par l'intermédiaire d'un distributeur étanche. Le mélange continue de réagir; le glycol est extrait sous pression réduite et/ou par entraînement. Le temps de séjour dans cette vis peut être compris entre 10 minutes et 2 heures. La sortie du produit terminé peut s'effectuer par un extracteur étanche. Le téréphtalate obtenu peut alors être stocké ou, dans le cas d'un téréphtalate de métal alcalin, mis en solution aqueuse pour une purification ultérieure. Selon une forme de réalisation particulière du procédé de l'invention, l'amorçage ou initiation de la réaction de saponification est effectué dans un malaxeur-extrudeur à double vis co-rotatives et la réaction est achevée dans un malaxeur à poudre, pouvant être à vis ou à bras en "Z", sous un courant de gaz inerte pour entraîner le glycol formé, qui sera condensé en dehors du réacteur.

L'élimination, en continu et de préférence sous pression réduite, du glycol formé au cours de la saponification constitue une autre amélioration selon l'invention: elle permet d'obtenir une saponification plus complète que suivant les modes opératoires de l'art antérieur, et d'augmenter la vitesse de réaction. On obtient, de cette façon, une conversion de polytéréphtalate en sel de l'acide téréphtalique égale ou supérieure à 95 pourcents en poids.

Contrairement aux procédés de l'art antérieur, qui conduisent à l'obtention de solutions diluées de téréphtalates de métaux alcalins ou alcalino-terreux, le procédé selon l'invention permet l'obtention de téréphtalates à l'état concentré, sous la forme d'un solide poudreux, plus facilement stockable et transportable.

Un autre avantage majeur du procédé selon l'invention réside dans sa moindre sensibilité à la présence éventuelle d'autres polymères tels que des polyoléfines ou du polychlorure de vinyle, en mélange avec le polytéréphtalate. Par conséquent, le procédé de l'invention convient particulièrement bien pour le recyclage de déchets de PET provenant du broyage ou déchiquetage de bouteilles ou flacons usagés.

Lors de la saponification de déchets de polytéréphtalates issus d'emballages, films, fibres ou bouteilles, diverses autres impuretés peuvent être présentes, telles que des charges, des additifs, des colorants, des revêtements, des étiquettes, etc. Le procédé de l'invention permet de séparer toutes ces impuretés, en dissolvant le produit de saponification dans l'eau et en filtrant la solution aqueuse, éventuellement avec un ou plusieurs adsorbants, avant d'acidifier la solution aqueuse pour précipiter l'acide téréphtalique.

Les exemples suivants sont donnés à tire d'illustration et ne limitent en rien la présente invention.

### EXEMPLE 1

Dans un malaxeur-extrudeur de 5" X 36" (15kw) fabriqué par la société AOUSTIN sous licence READCO, et comportant deux vis sans fin tournant dans le même sens et un système de chauffage du corps du malaxeur par circulation d'huile thermostatée, on ajoute en continu, à l'aide de deux doseurs volumétriques, 56 kg/h de copeaux de PET provenant du déchiquetage de films (taille moyenne des particules voisine de 4 X 1,5 cm; densité apparente voisine de 0.10) et 28 kg/h de paillettes de soude caustique de qualité technique, ces débits permettant de maintenir constamment un rapport pondéral PET/Na OH voisin de 2. La température de l'huile circulant dans la double enveloppe du malaxeur est comprise entre 150 et 160°C au démarrage et elle est abaissée à 120-130°C dès que le régime d'équilibre est atteint. La vitesse de rotation de la double vis est de 70 tours/minutes. Dans ces conditions, le temps moyen de séjour de la matière au sein du malaxeur est estimé à 6 minutes. Un prélèvement effectué sur le produit issu du malaxeur-extrudeur indique un taux de saponification supérieur à 60 pourcents.

Ce produit est ensuite dégazé sous pression réduite (10mmHg) pour éliminer l'éthylène glycol formé et il est maintenu à une température comprise entre 80 et 130°C durant 30 minutes par passage sur un tapis dans un tunnel chauffant.

On obtient de cette manière un solide poudreux constitué essentiellement de mono et di-téréphtalate sodiques. Le taux final de saponification est supérieur à 97 pourcents, alors que la totalité du PET mis en oeuvre est transformée.

### EXEMPLE 2

Dans le même appareillage que celui de l'exemple 1, et selon une procédure similaire, on traite 235 kg/h de déchets de PET broyés sous la forme de grains fins (quelques mm) de densité apparente voisine de 0,6 et 110 kg/h de soude caustique, sous la forme de pastilles. Dans ces conditions, toutes choses étant égales par ailleurs à l'exemple 1, le temps de séjour de la matière au sein du malaxeur est inférieur à 2 minutes. le taux de saponification obtenu dans ces conditions est voisin de 40 pourcents.

Le produit sortant du malaxeur-extrudeur à double vis corotatives est introduit ensuite dans un malaxeur à simple vis sans fin, d'une longueur de 4,5 mètres, maintenu à une température voisine de 150°C et balayé par un courant d'azote afin d'entraîner les vapeurs d'éthylène glycol formé qui sont, par ailleurs, condensées et récupérées.

Le solide poudreux produit est versé dans un excès d'eau de manière à obtenir une solution aqueuse diluée (par exemple 140 g/l) de téréphtalates sodiques. Cette solution est passée dans une colonne contenant du charbon actif; elle est ensuite acidifiée à l'acide sulfurique pour obtenir un pH voisin de 2,5 à 3. L'acide téréphtalique qui précipite est filtré et lavé à l'eau, puis séché.

La conversion du PET en acide téréphtalique est supérieure à 97 pourcents et la pureté de l'acide obtenu est supérieure à 99,5 pourcents, cet acide étant, en tout état de cause, utilisable comme acide de qualité-polymère.

### EXEMPLE 3

Comme dans les exemples 1 et 2, on traite 38,6 kg/h de potasse caustique et 54 kg/h de copeaux de PET provenant du déchiquetage de bouteilles usagées et contenant de l'ordre de 10 pourcents en poids de polyéthylène et de polychlorure de vinyle. La vitesse de rotation des vis est égale à 200 tours/minutes.

Le taux de saponification obtenu dans ces conditions, toutes choses étant égales par ailleurs, et calculé par rapport au poids de PET mis en jeu, est supérieur à 50 pourcents à la sortie du malaxeur-extrudeur à double vis et supérieur à 95 pourcents après un traitement selon le même mode opératoire que celui de l'exemple 2.

### EXEMPLE 4

2000 kg de polyester PET broyés en provenance de films sont mélangés à froid dans un mélangeur à ruban, avec 926 kg de soude à 98 pourcents minimum soit environ 10 pourcents de plus que la quantité stoechiométrique.

Le mélange des réactifs par l'intermédiaire d'un doseur alimente un malaxeur extrudeur à double vis pré-chauffé à 150°C, la sortie est reliée par l'intermédiaire d'un obturateur rotatif à une vis d'Archimède double dans une cuve fermée contrôlée thermiquement, dans laquelle le produit séjourne pendant 1/2 heure - l'ensemble est mis sous vide - la sortie de la cuve passe par un obturateur étanche de façon à maintenir le vide dans l'enceinte.

Le doseur du mélange de départ est mis en route; dès que le malaxeur extrudeur a atteint la température de 150°C, la réaction s'effectue et la circulation d'huile est réglée de telle façon que la température n'excède pas 150°C. Dès que la vis sans fin est alimentée, on applique un vide de l'ordre de 20 mm de Hg. Le glycol distille et le téréphtalate de sodium se transforme en une poudre très fluide, non collante, exempte de glycol. Ce glycol est récupéré par condensation et est d'une pureté suffisante pour une utilisation dans les fluides de refroidissement.

Les rendements observés par rapport au PET contenu dans le produit de départ :

| | | |
|---|---|---|
| Téréphtalate de sodium extrait | 2150 Kg | (Rt 98 pourcents). |
| Ethylène glycol | 601 Kg | (Rt 93 pourcents). |

## Revendications

1. Procédé de récupération d'un téréphtalate de métal alcalin ou alcalino-terreux à partir d'une composition comprenant principalement un polytéréphtalate d'alkylène, caractérisé en ce que
a) on mélange la compositon de départ avec un hydroxyde de métal alcalin ou alcalino-terreux pratiquement anhydre en l'absence de solvant;
b) on chauffe le mélange obtenu, ledit mélange étant au moins en partie à l'état fondu, de manière à amorcer la saponification du polytéréphtalate d'alkylène et à en permettre la poursuite sans chauffage ultérieur;
c) on élimine l'alkylèneglycol formé au cours de la réaction de saponification sous forme gazeuse; et
d) on recueille le téréphtalate de métal alcalin ou alcalino-terreux sous la forme d'un produit poudreux issu de la réaction de saponification.

2. Procédé selon la revendication 1, caractérisé en ce que l'ensemble des opérations est effectué en discontinu ou en continu.

3. Procédé selon l'une des revendications 1 et 2, caractérisé en ce que la réaction de saponification est amorcée dans un réacteur utilisant l'effet d'hystérésis et l'élévation subséquente de la température du mélange.

4. Procédé selon l'une des revendications 1 à 3, caractérisé en ce l'on fait passer le mélange de départ dans un réacteur-malaxeur-extrudeur, où il subit un transfert d'énergie calorifique par effet d'hystérésis, la réaction de saponification ainsi amorcée étant poursuivie à l'intérieur ou en dehors dudit réacteur-malaxeur-extrudeur.

5. Procédé selon l'une des revendications 1 à 4 caractérisé en ce que l'on amorce la réaction de saponification en introduisant un mélange solide de polytéréphtalate d'alkylène et d'hydroxyde de métal alcalin ou alcalino-terreux dans un réacteur-malaxeur-extrudeur à double vis corotatives ou non.

6. Procédé selon l'une des revendications 1 à 5, caractérisé en ce que le mélange de départ alimente en continu un réacteur-malaxeur-extrudeur à double vis corotatives présentant un arrangement des palettes d'entrée, de mélange, d'avance, de cisaillement et de retard permettant une homogénéisation et un échauffement du mélange réactionnel suffisant pour amorcer le processus de saponification.

7. Procédé selon l'une des revendications 1 à 6, caractérisé en ce que la température d'amorçage de la réaction de saponification est comprise entre 60 et 250°C.

8. Procédé selon l'une des revendications 1 à 7, caractérisé en ce que l'alkylèneglycol formé durant la réaction de saponification est éliminé en continu.

9. Procédé selon l'une des revendications 1 à 8, caractérisé en ce que l'alkylèneglycol formé est éliminé par entraînement au moyen d'un gaz inerte et/ou par extraction sous pression réduite.

10. Procédé selon l'une des revendications 1 à 9, caractérisé en ce que l'alkylèneglycol formé est éliminé au moins en partie dans le réacteur-malaxeur-extrudeur.

11. Procédé selon l'une des revendications 1 à 10, caractérisé en ce que le polytéréphtalate d'alkylène est un polytéréphtalate d'éthylène (PET).

12. Procédé selon l'une des revendications 1 à 11, caractérisé en ce que le polytéréphtalate d'alkylène de départ est constitué de déchets d'emballages, de films, de fibres ou de bouteilles.

13. Procédé de récupération d'acide téréphtalique à partir d'une composition comprenant principalement un polytéréphtalate d'alkylène, caractérisé en ce que
a) on mélange le composition de départ avec un hydroxyde de métal alcalin ou alcalino-terreux pratiquement anhydre en l'absence de solvant;
b) on chauffe le mélange obtenu, ledit mélange étant au moins en partie à l'état fondu, de manière à amorcer la saponification du polytéréphtalate d'alkylène et à en permettre la poursuite sans chauffage ultérieur;
c) on élimine l'alkylèneglycol formé au cours de la réaction de saponification sous forme gazeuse; et
d) on recueille le téréphtalate de métal alcalin ou alcalino-terreux sous la forme d'un produit poudreux issu de la réaction de saponification ; et
e) on dissout le téréphtalate de métal alcalin ou alcalino-terreux dans de l'eau et on acidifie la solution aqueuse obtenue pour obtenir un acide téréphtalique de pureté élevée.

## Patentansprüche

1. Verfahren zur Rückgewinnung von Alkalimetall- oder Erdalkalimetall-Terephtalat aus einer im wesentlichen ein Alkylenpolyterephtalat enthaltenden Zusammensetzung, dadurch gekennzeichnet, daß
a) die Ausgangszusammensetzung mit einem praktisch wasserfreien Alkalimetall- oder Erdalkalimetallhydroxid in Abwesenheit von Lösungsmittel gemischt wird;
b) das erhaltene Gemisch erwärmt wird, wobei das Gemisch wenigstens teilweise in geschmolzenem Zustand ist, um die Verseifung des Alkylenpolyterephtalats in Gang zu setzen und deren Fortsetzung ohne späteres Erwärmen zu gestatten;
c) das im Laufe der Verseifungsreaktion gebildete Alkylenglycol in Gasform beseitigt wird; und
d) das Alkalimetall- oder Erdalkalimetallterephtalat in Form eines aus der Verseifungsreaktion hervorgegangenen pulverförmigen Erzeugnisses aufgefangen wird.

2. Verfahren nach Anspruch 1, dadurch gekennzeichnet, daß die Gesamtheit der Operationen diskontinuierlich oder kontinuierlich durchgeführt wird.

3. Verfahren nach einem der Ansprüche 1 und 2, dadurch gekennzeichnet, daß die Verseifungsreaktion in einem Reaktor unter Ausnutzung des Hystereseeffekts und der daraus folgenden Erhöhung der Temperatur des Gemischs in Gang gesetzt wird.

4. Verfahren nach einem der Ansprüche 1 bis 3, dadurch gekennzeichnet, daß das Ausgangsgemisch in einen Knet-Extruder-Reaktor gegeben wird, wo es einen Wärmeenergieübertrag durch Hystereseeffekt erfährt, wobei die so in Gang gesetzte Verseifungsreaktion innerhalb oder außerhalb des Knet-Extruder-Reaktors fortgesetzt wird.

5. Verfahren nach einem der Ansprüche 1 bis 4, dadurch gekennzeichnet, daß die Verseifungsreaktion in Gang gesetzt wird durch Einführen eines festen Gemischs von Alkylenpolyterephtalat und Alkalimetall- oder Erdalkalimetallhydroxid in einen Knet-Extruder-Reaktor mit zwei gleichsinnig oder nicht gleichsinnig drehenden Schnecken.

6. Verfahren nach einem der Ansprüche 1 bis 5, dadurch gekennzeichnet, daß das Ausgangsgemisch kontinuierlich einen Knet-Extruder-Reaktor mit zwei gleichsinnigen Schnecken versorgt, der eine Anordnung von Eingangs-, Misch-, Vortriebs-, Scher- und Verzögerungsplatten aufweist, die eine zum In-Gang-Setzen des Verseifungsprozesses ausreichende Homogenisierung und Erwärmung des Reaktionsgemischs gestatten.

7. Verfahren nach einem der Ansprüche 1 bis 6, dadurch gekennzeichnet, daß die Temperatur der In-Gang-Setzung der Verseifungsreaktion zwischen 60 und 250°C liegt.

8. Verfahren nach einem der Ansprüche 1 bis 7, dadurch gekennzeichnet, daß das während der Verseifungsreaktion gebildete Alkylenglykol kontinuierlich beseitigt wird.

9. Verfahren nach einem der Ansprüche 1 bis 8, dadurch gekennzeichnet, daß das gebildete Alkylenglykol durch Mitnahme mit Hilfe eines inertes Gases und/oder durch Extraktion unter verringertem Druck beseitigt wird.

10. Verfahren nach einem der Ansprüche 1 bis 9, dadurch gekennzeichnet, daß das gebildete Alkylenglykol wenigstens teilweise im Knet-Extruder-Reaktor beseitigt wird.

11. Verfahren nach einem der Ansprüche 1 bis 10, dadurch gekennzeichnet, daß das Alkylenpolyterephtalat ein Ethylenpolyterephtalat (PET) ist.

12. Verfahren nach einem der Ansprüche 1 bis 11, dadurch gekennzeichnet, daß das Ausgangs-Alkylenterephtalat durch Abfälle von Verpackungen, Folien, Fasern oder Flaschen gebildet ist.

13. Verfahren zur Rückgewinnung von Terephtalsäure aus einer im wesentlichen ein Alkylenpolyterephtalat enthaltenden Zusammensetzung, dadurch gekennzeichnet, daß
a) die Ausgangszusammensetzung mit einem praktisch wasserfreien Alkalimetall- oder Erdalkalimetallhydroxid in Abwesenheit von Lösungsmittel gemischt wird;
b) das erhaltene Gemisch erwärmt wird, wobei das Gemisch wenigstens teilweise in geschmolzenem Zustand ist, um die Verseifung des Alkylenpolyterephtalats in Gang zu setzen und dessen Fortsetzung ohne späteres Erwärmen zu gestatten;
c) das im Laufe der Verseifungsreaktion gebildete Alkylenglykol in Gasform beseitigt wird; und
d) das Alkalimetall- oder Erdalkalimetallterephtalat in Form eines aus der Verseifungsreaktion hervorgegangenen pulverförmigen Erzeugnisses aufgefangen wird; und
e) das Alkalimetall- oder Erdalkalimetallterephtalat in Wasser gelöst und die erhaltene wäßrige Lösung angesäuert wird, um Terephtalsäure hoher Reinheit zu erhalten.

## Claims

1. Method for recovery of alkali metal or alkaline-earth metal terephthalate from a composition principally comprising a polyalkylene terephthalate, characterised in that
a) the initial composition is mixed with a substantially anhydrous alkali metal or alkaline-earth metal hydroxide in the absence of a solvent ;
b) the mixture obtained is heated, said mixture being at least in part in a molten state, in a manner such as to stimulate the saponification of the polyalkylene terephthalate and to permit its continuation without further heating ;
c) the alkylene glycol formed during the saponification reaction is removed in gaseous form ; and
d) the alkali metal or alkaline-earth metal terephthalate is recovered in the form of a powdery product.

2. Method according to claim 1, characterised in that all the operations are carried out non-continuously or continuously.

3. Method according to one of claims 1 and 2, characterised in that the saponification reaction is stimulated in a reactor using the effect of hysterisis and the subsequent increase in the temperature of the mixture.

4. Method according to one of claims 1 to 3, characterised in that the initial mixture is passed into a kneader-extruder reactor where it is subjected to a transfer of thermal energy by the effect of hysterisis, whereby the saponification reaction thus stimulated is continued in or outside said kneader-extruder reactor.

5. Method according to one of claims 1 to 4, characterised in that the saponification reaction is stimulated by introducing a solid mixture of polyalkylene terephthalate and alkali metal or alkaline-earth metal hydroxide into a kneader-extruder with double screws corotating or not.

6. Method according to one of claims 1 to 5, characterised in that the initial mixture is continuously fed into a kneader-extruder reactor with double corotating screws with an arrangement of blades for entry, mixing, moving forward, shearing and delay, allowing homogenisation and heating of the reaction mixture sufficient to stimulate the saponification process.

7. Method according to one of claims 1 to 6, characterised in that the stimulation temperature for the saponification reaction is in the range of 60 to 250°C.

8. Method according to one of claims 1 to 7, characterised in that the alkylene glycol formed during the saponification reaction is continuously removed.

9. Method according to one of claims 1 to 8, characterised in that the alkylene glycol formed is removed by entrainment by means of an inert gas and/or by extraction under low pressure.

10. Method according to one of claims 1 to 9, characterised in that the alkylene glycol is removed at least in part in the kneader-extrader reactor.

11. Method according to one of claims 1 to 10, characterised in that the polyalkylene terephthalate is a polyethylene terephthalate (P ET).

12. Method according to one of claims 1 to 11, characterised in that the initial polyalkylene terephthalate is comprised of packaging, film, fibre or bottle waste.

13. Method for recovery of terephthalic acid from a composition principally comprising a polyalkylene terephthalate, characterised in that
a) the initial composition principally composed of polyalkylene terephthalate, is mixed with substantially anhydrous alkali metal or alkaline-earth metal hydroxide in the absence of solvent ;
b) the mixture obtained is healed, said mixture being at least in part molten, in such a manner as to stimulate the saponification of polyalkylene terephthalate and to permit its going on without any further heating ;
c) the alkylene glycol formed during the saponification reaction is removed in gaseous form ; and
d) the alkali metal or alkaline-earth metal terephthalate is recovered in the form of a powdery material produced by the saponification reaction.
e) the alkali metal or alkaline-earth metal terephthalate is dissolved in water and the aqueous solution obtained is acidified to obtain a terephthalic acid of high purity.
